(19) Europäisches Patentamt
European Patent Office
Office européen des brevets

(11) Publication number: 0 082 656
A2

(12) # EUROPEAN PATENT APPLICATION

(21) Application number: 82306634.5

(22) Date of filing: 13.12.82

(51) Int. Cl.[3]: C 07 D 501/04
//C07D501/08, C07D205/08,
C07D499/04, C07D498/04,
(C07D498/04, 263/00, 205/00)

(30) Priority: 21.12.81 US 333154
21.12.81 US 333156

(43) Date of publication of application:
29.06.83 Bulletin 83/26

(84) Designated Contracting States:
BE CH DE FR GB IT LI LU NL SE

(71) Applicant: ELI LILLY AND COMPANY
307, East McCarty Street
Indianapolis Indiana 46285(US)

(72) Inventor: Kukolja, Stjepan
12123 Castle Overlook
Carmel Indiana 46032(US)

(72) Inventor: Pfeil, Janice Lucile
6024 Crittenden
Indianapolis Indiana 46220(US)

(74) Representative: Hudson, Christopher Mark et al,
Erl Wood Manor
Windlesham Surrey GU20, 6PH(GB)

(54) **Preparation of cepham derivatives.**

(57) There is described the production of a 7-*epi*-3-exomethylenecepham, useful in the synthesis of antibiotic compounds, by a process which comprises reducing a sulfoxide of formula (II):

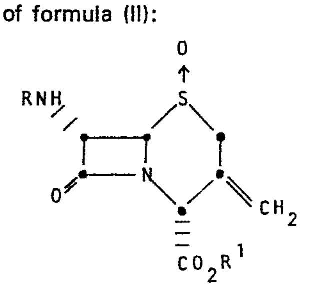

(II)

or acetoxymethyl derivative of formula (III):

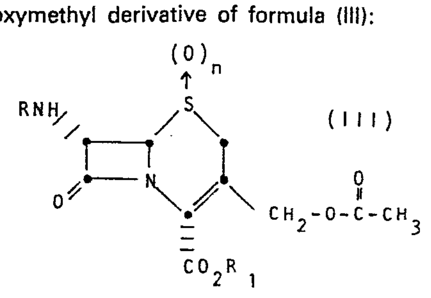

(III)

when n is 0 or 1 and R is hydrogen or an acyl group and $R^1$ is hydrogen, a carboxy-protecting group, or a cation.

EP 0 082 656 A2

PREPARATION OF CEPHAM DERIVATIVES

This invention relates to the synthesis of novel 7-*epi*-3-exomethylenecephams useful for the synthesis of antibiotically active cephalosporins. These new compounds possess a side chain at the 7-position of the cephalosporin nucleus in the α-configuration, rather than the β-configuration of natural cephalosporins.

Exomethylenecephams, useful as convenient intermediates, long have been known in pharmaceutical chemistry. *See*, for example, the article by Chauvette and Pennington, *J. Org. Chem 38*, 2994 (1973), in which the synthesis of such compounds is discussed.

Exomethylenecephams in which the side chain at the 7-position is in the α-configuration previously have not been available, mainly because the accepted procedures for epimerizing the compounds changed the exomethylenecepham to a 3-methylcephem. Epimerization of the cephalosporin and penicillin side chain is known to take place in the presence of strong bases; see, for example, Flynn, Cephalosporins and Penicillins, Academic Press, New York, 1972, pages 105-19. *See also* page 2996 of the Chauvette and Pennington article, where treatment with a strong base is shown to convert an exomethylenecepham to the 3-methylcephem.

According to the invention there is provided a process for preparing a 7-*epi*-exomethylenecepham of the formula (I):

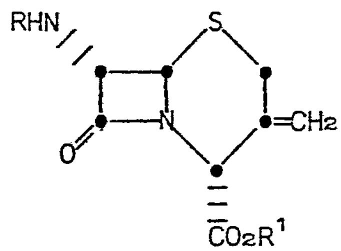

(I)

wherein R is hydrogen or an acyl group, and $R^1$ is hydrogen, a carboxy-protecting group, or a cation which process comprises reducing a sulfoxide of formula (II):

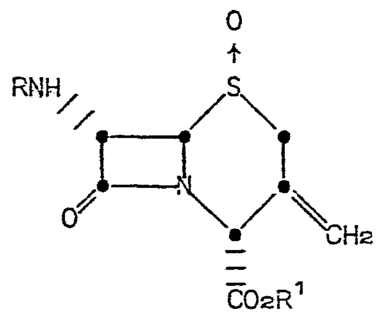

(II)

or acetoxymethyl derivative of formula (III):

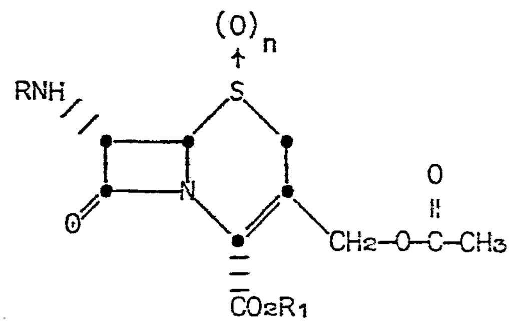

(III)

where n is 0 or 1 and R and $R^1$ are as defined above.

The reduction of the sulfoxide of formula (II) can be accomplished by electrolytic means or by reaction with an acyl halide, and a $C_{2-5}$ alkene. Similarly, the acetoxymethyl derivative of formula (III) may be reduced electrolytically.

All percentages, ratios, concentrations and the like are stated in weight units unless otherwise described and all temperatures are stated in degrees Celsius.

In the formula above, the group $R^1$ completes an acid or a salt, or is a carboxy-protecting group forming an ester. Such forms of cephalosporin compounds are conventional. In the context of this invention, the compounds in which $R^1$ is a carboxy-protecting group are preferred. The compounds where $R^1$ is a salt-forming cation, and the acids wherein $R^1$ is hydrogen, are also valuable. Particularly useful cations include such commonly used salt-forming moieties as sodium, potassium, lithium or ammonium.

In the antibiotic art, the term "carboxy-protecting group" refers to any suitable group used to block or protect the cephalosporin carboxylic acid functionality while reactions involving other functional sites are carried out. Such carboxylic acid-protecting groups are noted for their ease of cleavage, for example, by hydrolytic or hydrogenolytic methods to the corresponding carboxylic acid. Examples of suitable carboxylic acid-protecting groups are <u>tert</u>-butyl, 1-methylcyclohexyl, benzyl, 4-methoxybenzyl, 4-nitrobenzyl, acetoxymethyl, 1-acetoxyethyl, pivaloyloxymethyl, 1-pivaloyloxyethyl, carboethoxymethyl, 1-carboethoxyoxyethyl, phthalidyl, 2-iodoethyl, 2-bromoethyl, benzhydryl, phenacyl, 4-halophenacyl, dimethylallyl, 2,2,2-trichloroethyl, methoxymethyl, tri($C_1$-$C_3$ alkyl)silyl and succinimidomethyl. Other known carboxylic acid-protecting groups are described by E. Haslam in "Protective Groups in Organic Chemistry," J. F. W. McOmie, Ed., Plenum Press, New York, 1973, Chapter 5. The nature of such groups is not critical; however, because of availability, ease of handling and other desirable properties, certain carboxylic acid-protecting groups are preferred. A preferred selection of carboxylic acid-protecting groups includes acetoxymethyl, 1-acetoxyethyl, pivaloyloxymethyl, 1-pivaloyloxyethyl, carboethoxyoxymethyl, 1-carboxyethoxyoxyethyl, phthalidyl, diphenylmethyl, nitrobenzyl, tert-butyl, methoxybenzyl, trichloroethyl, and methyl.

The group R in the above general formula is hydrogen or an acyl group derived from a carboxylic acid, and, more particularly, is a group conventionally used in the cephalosporin art. This invention provides no new R groups, but makes use of those conventionally used by cephalosporin chemists in making antibiotically active compounds and intermediates for their synthesis. Preferred R groups, however, include those of the formula $R^2OC$, in which $R^2$ is hydrogen, $C_1$-$C_3$ alkyl, cyanomethyl, benzyloxy, 4-nitrobenzyloxy, t-butoxy, 2,2,2-trichloroethoxy, 4-methoxybenzyloxy, or the group $R^3$, in which $R^3$ is phenyl or phenyl substituted by 1 or 2 halogen, protected hydroxy, nitro, cyano, trifluoromethyl, $C_1$-$C_4$ alkyl or $C_1$-$C_4$ alkoxy groups; or $R^2$ is a group of the formula $R^4$-$(O)_n$-$CH_2$-, in which $R^4$ has the

same meanings as $R^3$ above, or is 1,4-cyclohexadienyl, 2-thienyl or 3-thienyl; n is 0 or 1; provided that when n is 1, $R^4$ has the same meanings as $R^3$; or $R^2$ is a group of the formula $R^4$-CH(W)-, wherein $R^4$ has the same meanings as defined above, and W is a protected hydroxy or protected amino group.

A more preferred class of $R^2$ groups includes $C_1$-$C_3$ alkyl, phenyl, phenoxymethyl, benzyl, and phenyl substituted with $C_1$-$C_4$ alkyl, especially with methyl.

In the above definitions, the terms $C_1$-$C_3$ alkyl, $C_1$-$C_4$ alkoxy and $C_1$-$C_4$ alkyl have their usual meanings in the organic chemical literature, and include groups such as methyl, methoxy, ethyl, ethoxy, propyl, isopropoxy, isobutyl, and s-butoxy.

The term protected amino refers to an amino group substituted with one of the commonly employed amino-protecting groups such as t-butoxycarbonyl, benzyloxycarbonyl, 4-methoxybenzyloxycarbonyl, 4-nitrobenzyloxycarbonyl, 2,2,2-trichloroethoxycarbonyl and 1-carbomethoxy-2-propenyl. Other accepted amino-protecting groups such as are described by J. W. Barton in Protective Groups in Organic Chemistry, Chapter 2 will be recognized by organic chemists as suitable.

Similarly, the term protected hydroxy refers to groups formed with a hydroxy group such as formyloxy, 2-chloroacetoxy, benzyloxy, diphenylmethoxy, triphenylmethoxy, 4-nitrobenzyloxy, trimethylsilyloxy, phenoxycarbonyloxy, t-butoxy, methoxymethoxy and tetrahydropyranyloxy. Other accepted hydroxy-protecting groups, such as those described by C. B. Reese

in Chapter 3 of Protective Groups in Organic Chemistry are understood to be included.

The 7-*epi*-exomethylenecephams are clearly and fully described by the above discussion. However, a few typical compounds provided by the invention will be named, to assure that the reader fully understands the invention:

*t*-butyl 7-α-acetamido-3-exomethylenecepham-4-carboxylate

benzyl 7-α-butyramido-3-exomethylenecepham-4-carboxylate

4-methoxybenzyl 7-α-(2-methylpropionamido)-3-exomethylenecepham-4-carboxylate

1-methylcyclohexyl 7-α-cyanoacetamido-3-exomethylenecepham-4-carboxylate

4-nitrobenzyl 7-α-benzyloxyformamido-3-exomethylenecepham-4-carboxylate

acetoxymethyl 7-α-(4-nitrobenzyloxyformamido)-3-exomethylenecepham-4-carboxylate

1-acetoxyethyl 7-α-*t*-butoxyformamido-3-exomethylenecepham-4-carboxylate

*t*-butoxymethyl 7-α-(2,2,2-trichloroethoxyformamido)-3-exomethylenecepham-4-carboxylate

4-chlorobenzoylmethyl 7-α-amino-3-exomethylenecepham-4-carboxylate

2-iodoethyl 7-α-(4-methoxybenzyloxyformamido)-3-exomethylenecepham-4-carboxylate

2-bromoethyl 7-α-phenylformamido-3-exomethylenecepham-4-carboxylate

diphenylmethyl 7-α-(3-chlorophenylformamido)-3-exomethylenecepham-4-carboxylate

ethoxycarbonyloxymethyl 7-α-(4-formyloxyphenylformamido)-3-exomethylenecepham-4-carboxylate

methoxymethyl 7-α-(2-nitrophenylformamido)-3-exomethylenecepham-4-carboxylate

trimethylsilyl 7-α-(3-cyanophenylformamido)-3-exomethylenecepham-4-carboxylate

2,2,2-trichloroethyl 7-α-(4-trifluoromethylphenylformamido)-3-exomethylenecepham-4-carboxylate

phthalimidomethyl 7-α-(3-methylphenylformamido)-3-exomethylenecepham-4-carboxylate

succinimidomethyl 7-α-(4-propylphenylformamido)-3-exomethylenecepham-4-carboxylate

2-pivalolyloxyethyl 7-α-(3-isobutylphenylformamido)-3-exomethylenecepham-4-carboxylate

2,2,2-tribromoethyl 7-α-(4-methoxyphenylformamido)-3-exomethylenecepham-4-carboxylate

1-acetoxypropyl 7-α-(2-ethoxyphenylformamido)-3-exomethylenecepham-4-carboxylate

7-α-(3-_t_-butoxyphenylformamido)-3-exomethylenecepham-4-carboxylic acid

sodium 7-α-(3-chloro-4-fluorophenylformamido)-3-exomethylenecepham-4-carboxylate

potassium 7-α-(2-bromo-5-chloroacetoxyphenylformamido)-3-exomethylenecepham-4-carboxylate

lithium 7-α-[3,5-bis(_t_-butoxy)phenyl]formamido-3-exomethylenecepham-4-carboxylate

ammonium 7-α-(tetrazol-1-yl)formamido-3-exomethylenecepham-4-carboxylate

7-α-(4-iodo-3-nitrophenylformamido)-3-exomethylenecepham-4-carboxylic acid

ammonium 7-α-(4-phenoxycarbonyloxy-2-cyanophenylformamido)-3-exomethylenecepham-4-carboxylate

7-α-(3-nitro-5-cyanophenylformamido)-3-exomethylenecepham-4-carboxylic acid

2,2,2-trichloroethyl 7-α-(3,5-dinitrophenylformamido)-3-exomethylenecepham-4-carboxylate

2-bromoethyl 7-α-(3-nitro-4-trifluoromethylphenylformamido)-3-exomethylenecepham-4-carboxylate

sodium 7-α-(2-methyl-4-trifluoromethylphenylformamido)-3-exomethylenecepham-4-carboxylate

diphenylmethyl 7-α-(3,4-diethylphenylformamido)-3-exomethylenecepham-4-carboxylate

4-nitrobenzyl 7-α-amino-3-exomethylenecepham-4-carboxylate

7-α-([3,5-bis(isopropoxy)phenylformamido]-3-exomethylenecepham-4-carboxylic acid

trimethylsilyl 7-α-(2-bromo-5-*s*-butylphenylformamido)-3-exomethylenecepham-4-carboxylate

4-methoxybenzyl 7-α-(1,4-cyclohexadienylacetamido)-3-exomethylenecepham-4-carboxylic acid

phthalimidomethyl 7-α-(thien-2-ylacetamido)-3-exomethylenecepham-4-carboxylate

*t*-butyl 7-α-phenylacetamido-3-exomethylenecepham-4-carboxylic acid

7-α-phenoxyacetamido-3-exomethylenecepham-4-carboxylic acid

potassium 7-α-(4-chlorophenoxyacetamido)-3-exomethylenecepham-4-carboxylate

7-α-(2,4-diiodophenylacetamido)-3-exomethylenecepham-4-carboxylic acid

diphenylmethyl 7-α-(4-bromo-3-*t*-butoxyphenoxyacetamido)-3-exomethylenecepham-4-carboxylate

7-α-(3-chloro-2-nitrophenylacetamido)-3-exomethylenecepham-4-carboxylic acid

benzyl 7-α-(3-cyano-5-trifluoromethylphenoxyacetamido)-3-exomethylenecepham-4-carboxylic acid

acetoxymethyl 7-α-(2-cyano-4-iodophenylacetamido)-3-exomethylenecepham-4-carboxylate

ethoxycarbonyloxymethyl 7-α-(4-benzyloxy-3-ethylphenoxyacetamido)-3-exomethylenecepham-4-carboxylate

potassium 7-α-(3,4-dinitrophenylacetamido)-3-exomethylenecepham-4-carboxylate

4-methoxybenzyl 7-α-(3,4-dicyanophenoxyacetamido)-3-exomethylenecepham-4-carboxylate

7-α-(3-iodo-4-propoxyphenylacetamido)-3-exomethylenecepham-4-carboxylic acid

7-α-(2,4-dimethoxyphenoxyacetamido)-3-exomethylenecepham-4-carboxylic acid

2,2,2-trichloroethyl 7-α-(2-isopropyl-4-methylphenoxyacetamido)-3-exomethylenecepham-4-carboxylate

7-α-[2,4-bis(trimethylsilyl)phenoxyacetamido]-3-exomethylenecepham-4-carboxylic acid

4-nitrobenzyl 7-α-(4-formyloxyphenoxyacetamido)-3-exomethylenecepham-4-carboxylic acid

trimethylsilyl 7-α-(3-nitrophenoxyacetamido)-3-exomethylenecepham-4-carboxylate

potassium 7-α-(4-cyanophenylacetamido)-3-exomethylenecepham-4-carboxylate

lithium 7-α-(2-trifluoromethylphenoxyacetamido)-3-exomethylenecepham-4-carboxylate

7-α-(4-ethylphenylacetamido)-3-exomethylenecepham-4-carboxylic acid

7-α-(3-propoxyphenoxyacetamido)-3-exomethylenecepham-4-carboxylic acid

4-methoxybenzyl 7-α-[chloroacetoxy(1,4-cyclohexadienyl)acetamido]-3-exomethylenecepham-4-carboxylate

1-methylcyclohexyl 7-α-[t-butoxyformamido(1,4-cyclohexadienyl)acetamido]-3-exomethylenecepham-4-carboxylate

trimethylsilyl 7-α-[(thien-2-yl)(4-methoxybenzyloxyformamido)acetamido]-3-exomethylenecepham-4-carboxylate

7-α-[(thien-2-yl)trimethylsilyloxyacetamido]-3-exomethylenecepham-4-carboxylic acid

acetoxymethyl 7-α-[phenyl(4-nitrobenzyloxyformamido)acetamido]-3-exomethylenecepham-4-carboxylate

7-α-[phenyl(benzyloxy)acetamido]-3-exomethylenecepham-4-carboxylic acid

7-α-[benzyloxyformamido(4-bromophenyl)acetamido]-3-exomethylenecepham-4-carboxylic acid

7-α-[trimethylsilyloxy(4-trimethylsilyloxyphenyl)acetamido]-3-exomethylenecepham-4-carboxylic acid

4-methoxybenzyl 7-α-[t-butoxy(3-nitrophenyl)acetamido]-3-exomethylenecepham-4-carboxylate

acetoxymethyl 7-α-[(4-cyanophenyl)(2,2,2-trichloroethoxyformamido)acetamido]-3-exomethylene-cepham-4-carboxylate

7-α-[benzyloxyformamido(2-nitrophenyl)acetamido]-3-exomethylenecepham-4-carboxylic acid

7-α-[(4-trifluoromethylphenyl)(4-nitrobenzyl-oxyformamido)acetamido]-3-exomethylenecepham-4-carboxylic acid

7-α-[diphenylmethoxy(4-methylphenyl)acetamido]-3-exomethylenecepham-4-carboxylic acid

trimethylsilyl 7-α-[triphenylmethoxy(4-t-butylphenyl)acetamido]-3-exomethylenecepham-4-carboxylate

4-chlorobenzoylmethyl 7-α-[phenoxycarbonyloxy(3-ethoxyphenyl)acetamido]-3-exomethylenecepham-4-carboxylate

7-α-[methoxymethoxy(4-s-butoxyphenyl)acetamido]-3-exomethylenecepham-4-carboxylic acid

7-α-[t-butoxyformamido(2,4-difluorophenyl)-acetamido]-3-exomethylenecepham-4-carboxylic acid

7-α-[benzyloxyformamido[3,5-bis(trifluoromethyl)phenyl]acetamido]-3-exomethylenecepham-4-carboxylic acid

4-nitrobenzyl 7-α-[trimethylsilyloxy(2,5-diethylphenyl)acetamido]-3-exomethylenecepham-4-carboxylate

4-methoxybenzyl 7-α-[diphenylmethoxy(2-chloro-3-cyanophenyl)acetamido]-3-exomethylenecepham-4-carboxylate

4-methoxybenzyl 7-α-[formyloxy(3-formyloxy-4-nitrophenyl)acetamido]-3-exomethylenecepham-4-carboxylate

diphenylmethyl 7-α-[t-butoxyformamido(2-methoxy-4-trifluoromethylphenyl)acetamido]-3-exomethylenecepham-4-carboxylate

The most preferred group of compounds of this invention are those wherein R represents one of the following:

a) hydrogen,
b) phenoxyacetyl,
c) phenylacetyl, or
d) 4-methylphenylformyl;

and $R^1$ represents one of the following:

a) hydrogen,
b) 4-nitrobenzyl,
c) 4-methoxybenzyl,
d) diphenylmethyl, or
e) 2,2,2-trichloroethyl.

The 7-epi-exomethylenecephams can be made by various processes. The preferred process is the electrolytic reduction of the corresponding 3-acetoxymethyl epi configuration cephem, or its sulfoxide. Examples 5 and 6 below illustrate this process. The electrolytic transformation of 3-acetoxymethyl cephalosporins to 3-exomethylene compounds finds precedent in the art, as shown by U.S. Patents 3,792,995, of Ochiai et al., and 4,042,472, of Hall, and is the preferred process.

One should note that if the electrolytic process is used on a starting compound having a nitrobenzyl protecting group, that protecting group will be cleaved, at least to some extent. The cleavage of the protecting group may be an advantage in some circumstances; if not, it can readily be avoided by merely using a different protecting group.

When the starting compound for the electrolytic reduction is a sulfoxide, the electrolysis reduces the 1-oxide to the desired sulfide form, as in Ex. 6 below.

Although the electrolytic cells used are the conventional types now known in the electrochemical art, a discussion of electrolytic cells, as they presently apply, will be given.

An electrolytic cell of the type used for electrolytic reductions has a working electrode, sometimes called the cathode, at which the reduction takes place. The working electrode is maintained at a potential which is negative with respect to the auxiliary electrode, or anode, at which only electrolyte reactions should take place. A reference electrode is usually used, also. The reference electrode, at which no reactions should take place, supplies a reference point from which the potential of the working electrode is measured. A typical and frequently-used reference electrode is the saturated calomel electrode; others are the mercury/mercuric oxide electrode and the silver/silver chloride electrode. The reference electrode is electrically connected to the working fluid through a conductive bridge or a porous junction.

Often, cells are divided into compartments, so that each of the electrodes is immersed in fluid which physically is separated from the fluids of the other compartments, but is electrically connected to them. Such division of the cell is optional in the context of the present invention, unless the compound to be reduced bears a group which can be electrically oxidized. The oxidizability of the starting compound may be readily determined by running a voltammogram on the auxiliary electrode in a positive direction with respect to the reference electrode. The presence of inflection points indicates that one or more oxidizable groups are present and that a divided cell is necessary, so that the auxiliary electrode (anode) is physically separated from the working fluid which contains the compound.

The arrangement of electrolytic cells, the construction of electrodes, and the materials which may be effectively used as dividers are all part of the common knowledge of the electrochemical art, and may easily be learned by reference to text books and journal articles. Particularly useful text books which may be mentioned include Organic Electrochemistry, M. M. Baizer, Editor, Marcel Dekker, Inc., New York (1973), and Technique of Electroorganic Synthesis, N. L. Weinberg, Editor, John Wiley and Sons, New York (1974).

Working electrodes for use in the process of this invention are made of carbon, mercury, tin, aluminum, copper, lead, chromium, zinc, nickel or

cadmium. The preferred working electrodes are mercury, silver and lead. The electrodes should be rather highly purified, as is normally the case in electrochemistry. The form of the electrode is not important; it may be solid sheet, gauze or cloth, a basket of shot, or a fluidized bed of particles. The electrode may also be made of an inert substrate plated with the electrode metal, or it may be made in the form of a sheet of the electrode composition, wrapped with gauze of the same composition to increase the electrode area.

The auxiliary electrode (anode) does not participate in the reductive process, and so it may be made of any suitable substance which is not attacked by the oxidative side of the electrolytic process. Auxiliary electrodes are most often made of the noble metals, especially platinum, or of carbon. Platinum oxide, or platinum coated with platinum oxide, is the preferred anode composition. Lead oxide, silver oxide and such metallic oxides may be used also for auxiliary electrode compositions.

It is most effective to arrange the cell so that the distance between the auxiliary electrode and the working electrode is everywhere the same, and is as small as possible. The relationship is desirable in all electrolytic processes, to maximize current flow and minimize temperature rise caused by the resistance of the fluid to the flow of current.

The process is carried out in an acidic working fluid, which is made acid by the addition of an acid, preferably sulfuric acid or hydrochloric acid, or of a buffering mixture of salts, acids and bases.

The acid condition is necessary to give up protons to the reaction at the working electrode, and also to keep the working fluid acid, because the products are unstable in basic conditions.

If an undivided cell is used, the fluid in contact with both the working electrode and the auxiliary electrode will be the same. If the cell is divided, however, the working fluid undoubtedly will be different from the fluid in the auxiliary electrode compartment.

The working fluid used in this invention may be aqueous, organic or mixed. The organic portions of the working fluid may be either water-miscible or water-immiscible. If a mixed system is used, a water-miscible solvent is preferred so that the working fluid is a homogeneous solution. Suitable water-miscible organic solvents include the amides, especially dimethylformamide and dimethylacetamide, acetone, the water-miscible alkanols, such as methanol, ethanol and propanol, and tetrahydrofuran.

If a water-immiscible solvent is used in the working fluid, the choice of solvents is extremely broad because any solvent which is not reduced at the working electrode may be used. Especially desirable solvents include the halogenated solvents, such as dichloromethane, 1,1,2-trichloroethane, chloroform, chlorobenzene, 1,1,1-trichloroethane, as well as others. Some immiscible solvents which may be used include, as well as others, the ketones such as methyl ethyl ketone, methyl butyl ketone and methyl isobutyl

ketone; the aromatic solvents such as benzene, toluene and the xylenes; the alkanes such as pentane, hexane and the octanes; the alcohols such as phenol, the butyl alcohols, and ethers such as diethyl ether, diisopropyl ether and hexahydropyran.

An electrolyte may be used as well as the acid or salts which maintain the acidity of the working fluid. Such electrolytes are commonly used in the electrochemical art, and are preferably chosen from the class of quaternary ammonium salts. Useful electrolytes for this purpose include, among others, tetraethylammonium perchlorate, tetrabutylammonium perchlorate, benzotributylammonium chloride, benzyltriethylammonium bromide, benzyltriethylammonium chloride, methyltributylammonium iodide, tribenzylethylammonium p-toluenesulfonate.

If the process is to be carried out in a divided cell, the divider may be made of any of the materials commonly used in electrochemistry for the purpose. Especially useful dividers are made from ion exchange membranes, especially those which can pass cations. Useful dividers also may be made of finely porous substances such as ceramic membranes and sintered glass membranes. Such porous dividers may be made permeable to ions, but not to the fluids themselves by sealing the membranes with a conductive gel such as agar gel saturated with an ionic substance such as potassium sulfate.

When the auxiliary electrode occupies a cell compartment by itself, it is immersed in a conductive fluid. If the divider is a porous membrane, it is advisable to provide an auxiliary electrode fluid which is compatible with the working fluid, such as an aqueous solution of the mineral acid used in the working fluid. If the cell divider is porous only to ions, then the auxiliary electrode fluid may be any convenient conductive fluid, such as dilute aqueous solutions of ionizable salts and acids.

The temperature of the process is from about 0° to about 75°, preferably from about 0° to about 30°.

The potential of the working electrode, or the potential between the working electrode and the auxiliary electrode, may be controlled in various ways. The most effective and precise way to control the potential is to use a reference electrode, with its junction to the working fluid placed as physically close as possible to the working electrode. The desired potential for the process is determined from examination of a voltammogram of the system, and the potential between the working electrode and the auxiliary electrode is adjusted to give the desired constant potential between the reference electrode and the working electrode. This method of control is much more effective than control by the overall voltage between the working electrode and the auxiliary electrode, because that voltage depends on the condition of the dividing membrane, if such a membrane is used; the

concentration of the acid in the working fluid; and, the concentration of the compound to be reduced in the working fluid.

Similarly it is relatively inefficient to control the system by means of the current flow between the auxiliary electrode and the working electrode, because the current flow is directly dependent on the concentration of the compound to be reduced, as well as upon the physical condition of the electrodes and of the divider. When, however, an individual reduction has been thoroughly studied and the relationship between current, time and concentration is known, controlled-current electrolysis can be used for production of repeated batches.

Thus, the best way to control the system is by the potential between a reference electrode and the working electrode. This control is provided most conveniently by an automatic instrument which constantly senses that potential and adjusts the voltage between the working electrode and auxiliary electrode accordingly. Such instruments are now readily available; one maker of them is Princeton Applied Research, Inc., Princeton, N.J., U.S.A.

As has been briefly discussed above, the potential for operating the process with any given combination of electrodes, working fluid and compound is determined according to the routine method of the electrochemical art, by running a voltammogram of the system.

To name a precise potential range for the operation of the process is impossible because the potential for every system necessarily will be different. The potential, however, of the working electrode for reductions according to this process is from about -1 volt to about -2 volts, relative to a saturated calomel reference electrode, in the majority of systems.

Electrolytic cells usually require good agitation, and this process is typical in this respect. Agitating the working fluid to keep the surface of the electrode thoroughly swept so that a fresh supply of compound to be reduced is constantly supplied to the working electrode is advised. Further, when a water-immiscible solvent is used in the working fluid, sufficient agitation of the fluid, to keep the two phases of the working fluid intimately mixed in the form of fine droplets, is necessary.

Generally, the electrochemical art long has known that electrolytic processes are performed better and easier in flow cells than in batch electrolytic cells. A flow cell is an electrolytic cell arranged for the constant passage of the working fluid through the cell. The cell volume may be quite small, and the current density rather high, to achieve the desired extent of reaction in a single pass through the cell, or the flow rate may be lower and the volume larger, with the expectation that a number of passes through the cell will be necessary. If used, the flow cell is operated continuously with no interruptions for filling

and emptying the cell, and the associated operations of product isolation and temperature control are carried on outside the cell.

Flow cells are set up just as are batch cells except for the need to provide means for the entry and exit of the working fluid. A flow cell may be divided, if necessary, in the usual manner. Often, designing a flow cell with the electrodes spaced advantageously close to each other is possible because the agitation of the working fluid is provided by its own flow velocity and mechanical agitation of the cell is unnecessary. For example, a flow cell is often built in the form of a plate-and-frame filter press, with the electrodes in sheet form, clamped between the frames.

The concentration of the compound to be reduced in the working fluid is widely variable and is limited only by the solubility of the compound. Of course, using relatively high concentrations, in order to obtain the maximum effect from the solvents used in the process is most economical. Workup of the fluid and isolation of the product, however, is frequently more difficult when highly concentrated working fluids are used. Accordingly, concentrations of compound in the working fluid higher than about 20% weight/volume are not practical.

The 3-acetoxymethyl 7-epi configuration cephem compounds used in the process are obtained by methods long known to cephalosporin chemists. Kim and McGregor, J. Antibiotics 27, 831-33 (1974) show the

epimerization of a natural configuration 3-acetoxymethylcephem ester, and show that the 7-amino group remained in the α-configuration while the side chain was deacylated and reacylated, and while the ester group was removed. Thus, their epimerization process, consisting of reaction at ambient temperature with diisopropylamine in tetrahydrofuran of the Schiff's base of the formula

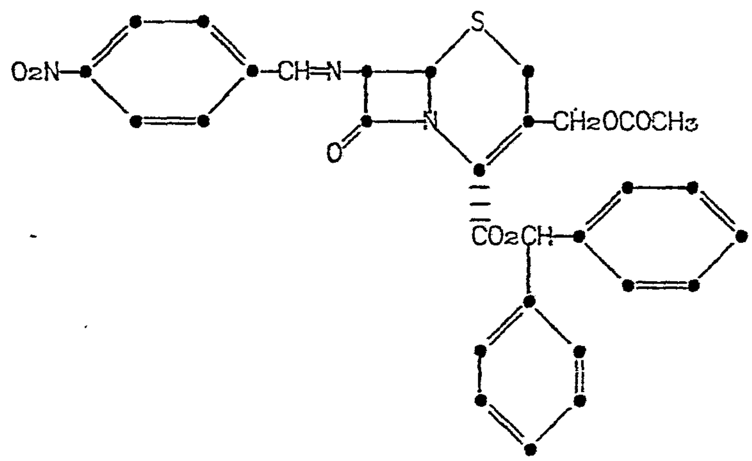

is useful to prepare any desired 7-epi configuration 3-acetoxymethyl starting material, by use of a starting compound having the desired R and $R^1$ groups, or by deacylating, acylating, deesterifying or esterifying according to methods commonly used in cephalosporin chemistry.

Another source of 7-epi configuration 3-acetoxymethyl starting compounds is shown by Sassiver and Shepherd, Tet. Let., 3993-96 (1969), who epimerized the 9-fluorenyl ester of 3-acetoxymethyl-7-β-(thien-2-yl)-3-cephem-4-carboxylic acid, 1-oxide, to the

7-*epi* configuration by simple contact with triethylamine in dimethyl sulfoxide at 50°. The process of Sassiver and Shepherd, thus, will provide any of the 3-acetoxymethyl starting compounds in the 7-*epi* configuration, combining their process with the deacylation, acylation, deesterification and esterification processes commonly used. The sulfoxides provided by Sassiver and Shepherd are most easily reduced to the sulfide form, if desired, by use of Hatfield's acyl bromide/bromine scavenger process, as taught in U.S. Patent 4,044,002. The reduction preferably is carried out with acetyl bromide and a $C_2$-$C_5$ alkene, such as ethylene, in an inert organic solvent at -25° to 50°.

Another method of obtaining 7-*epi*-exomethyleneсephams starts with 4-nitrobenzyl 6β-phenoxyacetamidopenam-3-carboxylate, 1-oxide, which is epimerized to the 6α-configuration by treatment with a silylating agent and a strong base. Most preferably, the natural configuration sulfoxide is treated with a large excess of triethylamine and a large excess of trimethylchlorosilane at moderately low temperatures in the range of about 0° to ambient temperature. Use of about 5 moles of triethylamine per mole of penicillin sulfoxide, and to carry out the process in an inert organic solvent such as dichloromethane is preferred. The epimerization is illustrated by a Preparation below.

The 6-*epi* penicillin oxide is ring-opened to form the sulfinyl chloride of the formula

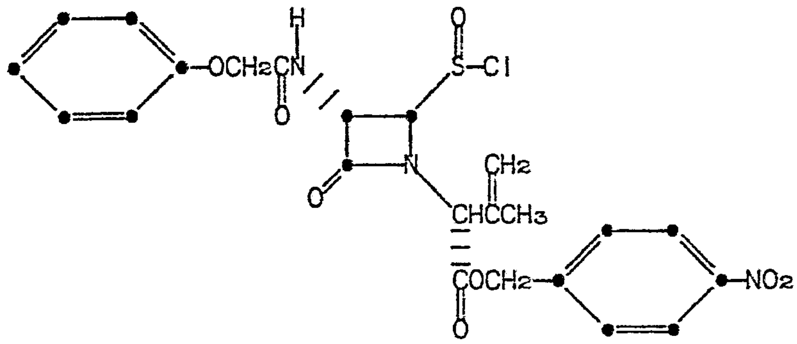

which is then ring-closed to form a 7-*epi* exomethylenecepham oxide and then reduced to a 7-*epi*-exomethylene cepham.

This process is carried out in benzene, toluene or a mixture of benzene and toluene. It is most preferred to use, as the reaction medium, a mixture of equal volumes of benzene and toluene, such that an ambient pressure reflux temperature of about 92° is obtained. The temperature range for the process is from about 80° to about 110°, more preferably from about 85° to about 94°.

The chlorinating agent used to prepare the sulfinyl chloride is N-chlorophthalimide, of which a moderate excess should be used. From about 1.1 to about 1.5 moles of the chlorinating agent per mole of penicillin sulfoxide are used, most preferably from about 1.1 moles to about 1.3 moles. The reaction time is from about 1 to about 3 hours.

To control the amount of free hydrochloric acid in the reaction mixture is very important, and, accordingly, a very effective acid scavenger is needed.

Such an acid scavenger is a polymer consisting of poly(4-vinylpyridine) cross-linked with about 2% to about 5% of divinylbenzene. The use of such polymers as acid scavengers is taught in full detail by U.S. Patent 4,289,695, of Chou. The polymer is used in particulate form, having a size distribution from about 20 to about 120 microns, although some part of the particles may be smaller and larger, and a relatively large amount of the polymer should be used, in the range of from about 0.8 gram to about 2 grams per gram of starting penicillin sulfoxide.

To carry out this process under substantially anhydrous conditions, and to exclude all basic contaminants from the reaction mixture is most important. The maintenance of this condition is a problem, because the epimerization process which forms the starting compound is carried out in a strong base, and because the purified 6-epi penicillin sulfoxide starting material retains a molecule of water in hydrate form. Thus, it is critical thoroughly to purify and wash the 6-epi penicillin sulfoxide to remove substantially all of the triethylamine or other strong base used in the epimerization.

The molecule of water of hydration attached to the penicillin sulfoxide cannot be removed by ordinary drying processes, and, therefore, must be removed from the reaction mixture before the process starts, or else very quickly thereafter. Heating the solvent and the polymer to the reflux temperature, and then adding the penicillin sulfoxide while heating is

continued is preferred. The sulfoxide then dissolves, the water of hydration leaves the reaction mixture in the form of an azeotrope, and can be removed from the reflux returning to the reaction vessel by a conventional water trap, or by other means such as passing the reflux through some water-absorbing medium such as calcium oxide. When all of the water substantially has been removed, the N-chlorophthalimide is added and the reaction starts.

Alternatively, if adding the N-chlorophthalimide and the penicillin sulfoxide at the same time or adding the penicillin sulfoxide as the last addition to the reaction mixture is desired, the mixture must be refluxing vigorously before the penicillin sulfoxide is added, so that its water of hydration will be thoroughly and quickly removed as fast as it dissolves.

Further alternatively, if avoiding reflux of the reaction mixture is necessary, it can be dried in the reaction vessel by other known means, such as by the addition to the reaction mixture of molecular sieves, water-absorbing inorganic salts, phosphorus oxides or other known dehydrating agents. Such expedients, however, are not preferred and the use of azeotropic distillation, as described above, is the preferred method for obtaining the necessary substantially anhydrous conditions.

The sulfinyl chloride so obtained is ring-closed according to the process of U.S. Patents 4,052,387 and 4,190,724. Thus, the epi sulfinyl chloride is reacted with a Lewis acid or proton acid Friedel-Crafts catalyst, or a metathetic cation-forming agent to form a complex. The preferred reagent is stannic chloride; other typical reagents include, e.g., zinc chloride, zinc bromide, titanium tetrachloride, methanesulfonic acid, trifluoroacetic acid, phosphoric acid, sulfuric acid, polyphosphoric acid, silver toluenesulfonate, silver perchlorate and the like. Forming the complex is preferred in a dry organic solvent, most preferably toluene or benzene, in the presence of an oxo ligand. Diethyl ether is the preferred ligand, and acetone, diethyl ketone, tetrahydrofuran, dioxane, cyclohexanone, triphenylphosphine oxide and the like are further typical ligands. The resulting insoluble complex cyclizes to the exomethylenecepham in the solid state, and is then decomposed with a hydroxy-containing compound, preferably methanol, to provide the desired product. The examples below further illustrate the process.

The 7-epi exomethylenecepham sulfoxide so obtained is reduced to a 7-epi-exomethylenecepham by the acyl bromide/bromine scavenger technique of Hatfield, U.S. Patent 4,044,002, described above.

Still further, 7-epi-exomethylenecephams can be prepared by the general process of U.S. Patents 4,052,387, 4,081,440 and 4,190,724. Accordingly, a 6-epi penicillin sulfoxide having the desired R and $R^1$ groups is reacted with an N-chlorohalogenating agent at 75° to 135° under anhydrous conditions. Halogenating agents include ureas, amides, imides, urethans, sulfonamides, and the like, and most preferably the imides

such as N-chlorosuccinimide, N-chloroglutarimide and especially N-chlorophthalimide. The reaction is performed in an inert organic solvent, preferably an aromatic solvent, and a non-alkaline acid scavenger is preferably included in the reaction mixture; epoxides and alkaline metal oxides, and especially the vinylpyridine polymer discussed above, are suitable scavengers.

The epi sulfinyl chloride so produced is cyclized as discussed above to form the 7-epi exomethyleneecepham sulfoxide, and reduced as discussed above to form the desired product of this invention.

Compounds in which $R^1$ is hydrogen may be recovered and isolated as the acid, or as a salt, in the usual way. That is, when a salt is desired, the compound is isolated from a solution of the appropriate base in water or a suitable solvent such as a low molecular weight ketone or alcohol, or an aqueous ketone or alcohol.

Compounds in which R is hydrogen are best obtained by deacylating a corresponding compound in which R is an acid residue. The deacylation may be performed by conventional methods, such as the processes taught by U.S. Patents 3,697,515, 3,875,151, 3,957,771, 4,021,426, 3,234,223. In general, such compounds are deacylated by reacting the 7-α-amido compound with a halogenating agent which can form an imido halide, such as an acid halide, especially phosphorus pentachloride, in the presence of a tertiary amine. The imido halide is converted to an imido ether

by reaction with an alcohol, especially a lower alkanol such as methanol, and hydrolyzed to form the 7-α-amino compound with water, an alcohol or an aqueous alcohol.

The 7-*epi*-exomethylenecephams are used best in a process which is described more fully below. In this process 7-*epi* configuration exomethylenecephams react first with molecular chlorine and then with a tri(alkyl or phenyl)phosphine to prepare an oxazolinoazetidinone of the formula

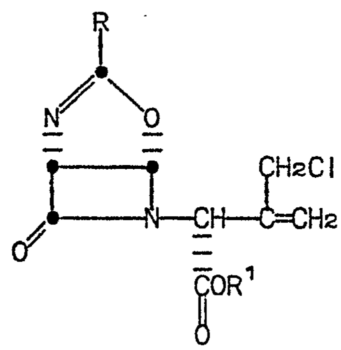

The above group of compounds are known intermediates used in the synthesis of oxa-β-lactam antibiotics, as shown by publications such as U.S. Patent 4,220,766 and South African Patent 77/7646, both of Shionogi and Company. The Preparations below further illustrate the synthesis of the oxazolinoazetidinones from the compounds of this invention.

The process is carried out in an inert organic solvent, preferably a halogenated organic solvent such as dichloromethane, 1,1,2-trichloroethane, chloroform, 1,2-dichloroethane, chlorobenzene, the

various dichlorobenzenes, 1,2-dibromoethane, and the like. Solvents can also be chosen from the aromatics such as benzene, toluene and the xylenes.

The chlorination step of the process is carried out at a very low temperature such as from about -100° to about -20°, preferably from about -100° to about -60°. The reaction with the phosphine is carried out at more moderate temperatures from about -50° to about 50°, preferably from about 0° to the ambient temperature. Ambient temperature means those temperatures which are normally encountered in occupied buildings, such as from about 15° to about 35°.

It is important to carry out the process under substantially anhydrous conditions. Drying the solvent very carefully, as by contact with molecular sieves, or by azeotropic distillation, if the solvent lends itself to it, is advisable. Because of the very low temperatures of the process, condensation in the vessel is a possible problem, and, therefore, must be avoided by use of drying tubes or columns.

In the preferred practice, the 7-epi exomethylenecepham is dissolved in an appropriate amount of solvent. The concentration of the reactant is not critical, but may be chosen for convenience in a given set of circumstances. Molecular chlorine is then added, either by bubbling through the solution, or as a solution in additional solvent. No unusual excess of chlorine is needed. Approximately 1 mole of $Cl_2$ per mole of starting compound is adequate; small excesses in the range of from a few percent to 20 percent will

increase the yield, and very large excesses, even up to 10X, are not harmful. The mixture is then stirred at constant temperature for a relatively brief period of time until the reaction has gone to the desired degree of completion. An operator may wish to maximize the yield of the process by using relatively long reaction times, or to maximize throughput of product, by minimizing reaction times.

The phosphine is then added to the first reaction mixture. A molar amount of the phosphine, or a small to moderate excess as discussed above, may be used. It is preferred to warm the mixture after the addition of the phosphine to a higher temperature as described above, or alternatively to turn off the cooling and allow the mixture to warm naturally toward the ambient temperature.

The phosphines which may be used, among others, are commonly used substances well understood by process chemists. A few of them, however, will be mentioned to assure that there is no misunderstanding:

triphenylphosphine
tri(isopropyl)phosphine
dipropylmethylphosphine
diethylphenylphosphine
tri(isobutyl)phosphine
diethyl (*s*-butyl)phosphine
dimethyl(*t*-butyl)phosphine

The following Preparations and Examples are provided to assure that the reader fully understands the use of this invention.

The first two Preparations immediately below illustrate the process for preparing an epi configuration sulfinyl chloride, as discussed above.

Preparation 1

4-Nitrobenzyl 6-α-phenoxyacetamidopenam-3-carboxylate, 1-oxide

A 250 g. portion of 4-nitrobenzyl 6-β-phenoxyacetamidopenam-3-carboxylate, 1-oxide, was dissolved in 1750 ml. of dichloromethane, and was cooled to 0-5°. The temperature was held constant while 272.5 ml. of triethylamine was added over 15 minutes. The mixture was stirred for 15 minutes, and then 214 ml. of trimethylchlorosilane and 70 ml. of additional triethylamine were added, and the mixture was stirred for 5 hours, still at constant temperature. To the mixture was then added slowly 175 ml. of acetic acid, and the temperature was allowed to rise to 20-25°. It was then washed twice with 1000 ml. portions of water, and then with 1000 ml. of 5% aqueous sodium carbonate. Five hundred ml. of additional dichloromethane was added, and the water layers were combined and extracted twice with 250 ml. portions of dichloromethane. All of the organic layers were combined, and chilled overnight. The crude product was isolated by filtration, and dried to obtain 92.9 g. of the desired product. An additional 80.8 g. of crude product was obtained by evaporating the solvent from the filtrate, dissolving the resulting gum in 100 ml. of acetone, and crystallizing the product from it by

chilling, seeding and the addition of a small amount of water. The portions of product were recrystallized by dissolving each in 600 ml. of warm acetone, filtering the warm solution, chilling the filtrate, and washing the resulting crystals with dichloromethane. A total yield of 156.7 g. of recrystallized product was obtained, having a melting range of 123-125° and 122-124° in the two sections.

Preparation 2

4-Nitrobenzyl 2-(2-oxo-3-α-phenoxyacetamido-4-chlorosulfinylazetidino)-3-methyl-3-butenoate

To a 1000 ml. flask were added 500 ml. of benzene and 12.5 g. of poly(4-vinylpyridine)polymer, cross-linked with 5% of divinylbenzene, in the form of a powder substantially all of which passed a 120-mesh screen. The suspension was heated to the reflux temperature, and the reflux was returned to the flask through a water trap until no more water was collected. The suspension was then cooled slightly, and to it were quickly added 12.5 g. of the product of Preparation 1 and 5.5 g. of N-chlorophthalimide. The mixture was quickly heated back to the reflux temperature and heated at that temperature for 5 hours, while the reflux was returned through a water trap to the flask.

The mixture was then cooled to 0-5° and filtered, to obtain a benzene solution of the desired sulfinyl chloride, containing considerable starting compound. The presence of the desired product was

confirmed by nuclear magnetic resonance analysis on a 60 mHz instrument. The following features are characteristic: δ 1.80 (s, 3H, $-CH_3$); 4.50 (s, 2H, $-OCH_2-$); 5.05(s) and 5.05-5.30 (m, 3H total, H2 and $=CH_2$ of butenoate); 5.30 (s, 2H, $-CO_2CH_2-$); 5.77 (d, 1H, J = 2 cps, H4 of azetidine); 5.93 (dd, 1H, J = 2 cps, H3 of azetidine); 7.73 (bs, 1H, -NH-); 6.73-7.37 (m, 5H, phenoxy aromatic); 7.5-8.17 (2d, 4H, J = 9 cps, benzyl aromatic)

## Example 1

4-Nitrobenzyl 7-α-phenoxyacetamido-3-exomethylenecepham-4-carboxylate, 1-oxide

To the product solution above was added 1.6 ml. of diethyl ether and 5.85 ml. of stannic chloride, and the mixture was stirred for 16 hours at ambient temperature. The resulting complex was isolated by filtration and washed with hexane, and the washed filter cake was added to 75 ml. of methanol and agitated. After 30-45 minutes of stirring, the solution was placed in an ice bath and stirred there for 6 hours. The crystalline product was filtered and washed with methanol to obtain 4.6 g. of the desired 7-epi exomethylenecepham sulfoxide, m.p. 190.5-191.5°.

## Preparation 3

4-Nitrobenzyl 2-(2-oxo-3-α-phenoxyacetamido-4-chlorosulfinylazetidino)-3-methyl-3-butenoate

The process of Preparation 2 was followed, in general, except that the solvent was a mixture of

250 ml. of benzene, carefully azeotropically distilled to eliminate water, and 250 ml. of toluene. The reaction temperature was the reflux temperature of the mixture, 92°, and reflux was continued for 2 hours and 20 minutes. The resulting solution of the sulfinyl chloride, when analyzed by nuclear magnetic resonance analysis, was found to contain very little starting compound.

Example 2

4-Nitrobenzyl 7-α-phenoxyacetamido-3-exomethylenecepham-4-carboxylate, 1-oxide

The product solution from Preparation 3 was subjected to the process of Example 1, except that 100 ml. of methanol was used, to obtain 7.1 g. of the desired 7-epi exomethylenecepham sulfoxide, m.p. 185-189.5°C.

Preparation 4

4-Nitrobenzyl 2-(2-oxo-3-α-phenoxyacetamido-4-chlorosulfinylazetidino)-3-methyl-3-butenoate

The process of Preparation 3 was followed, on a scale twice as large, except that reflux was continued for 2 hours 45 minutes. Nuclear magnetic resonance analysis of the product solution showed that essentially all of the starting compound had been consumed.

Example 3

4-Nitrobenzyl 7-α-phenoxyacetamido-3-exomethylenecepham-4-carboxylate, 1-oxide

The process of Example 1 was followed using the product solution of Preparation 4 on a scale twice as large as Example 1, to obtain 14.6 g. of the expected product, m.p. 182-188° after drying.

Preparation 5

4-Nitrobenzyl 2-(2-oxo-3-α-phenoxyacetamido-4-chlorosulfinylazetidino)-3-methyl-3-butenoate

The process of Preparation 4 was followed again, except that the reflux time was only 2 hours 30 minutes. The resulting product solution was found by nuclear magnetic resonance analysis to contain very little starting compound.

Example 4

4-Nitrobenzyl 7-α-phenoxyacetamido-3-exomethylenecepham-4-carboxylate, 1-oxide

The process of Example 3 was followed again on the product solution of Preparation 5, except that 4.15 ml. of diethyl ether was used instead of 3.6 ml. A 14.9 g. portion of the desired product, m.p. 189-191° after drying, was obtained.

Preparation 6

4-Nitrobenzyl 7-α-phenoxyacetamido-3-exomethylenecepham-4-carboxylate

A 1.0 g. portion of 4-nitrobenzyl 7-α-phenoxy-acetamido-3-exomethylenecepham-4-carboxylate, 1-oxide,

was dissolved in 30 ml. of dichloromethane, and the solution was cooled to 0-5°. To it was added 1.06 ml. of 2-methyl-2-butene and 0.33 ml. of acetyl bromide, and the mixture was stirred at 0-5° for 90 minutes. The mixture was then quenched by the addition of a large amount of water, and the organic layer was separated. It was then washed twice with 20 ml. portions of water and once with saturated sodium chloride solution, and was dried over magnesium sulfate. The product was isolated by low pressure high performance liquid chromatography on a silica gel column, using a 1:1 mixture of acetonitrile and water as the eluant. The product-containing fraction was evaporated to dryness under vacuum to obtain a small amount of the expected product.

The next two Examples illustrate the electrolytic reduction of 3-acetoxymethyl starting compounds.

## Example 5

7-α-(4-methylphenylformamido)-3-exomethylenecepham-4-carboxylic acid

An electrolytic cell having a total volume of 50 ml. was set up with a toroidal mercury working electrode having an area of 14 $cm.^2$. The auxiliary electrode was a loop of platinum wire placed parallel to the surface of the working electrode and separated from it by a fine glass frit. The reference electrode was a saturated calomel electrode, with its junction placed very close to the surface of the working electrode.

The cell was charged with 50 ml. of 1-molar pH 4.0 McIlvaine buffer containing 876 mg. of 7-α-(4-methylphenylformamido)-3-acetoxymethyl-3-cephem-4-carboxylic acid.

An automatic potentiostat was used to control the potential between the working electrode and the reference electrode at -1.6 volt. The electrolysis was continued for 233 minutes while the temperature was controlled at 25°. The working fluid was then washed out of the cell with deionized water, made acid to about pH 2, and extracted with ethyl acetate. The organic layer was dried and evaporated to dryness under vacuum to obtain 356 mg. of the desired product in impure form as identified by n.m.r. spectroscopy in $d_6$ acetone.

δ 2.37 (2, 3H, $-CH_3$); 3.37, 3.71 (AB, J = 14 Hz, 2H, H2 of thiazine); 5.23 (m, 5H, $=CH_2$, H4, H6, H7); 7.26, 7.84 (AB, J = 8Hz, 4H, aromatic)

## Example 6

7-α-(4-methylphenylformamido)-3-exomethylene-cepham-4-carboxylic acid

The process of Example 5 was used again in large part, with a sample of 406 mg. of the starting compound, in the 1-oxide form, in a total volume of 35 ml. of working fluid. The electrolysis was continued for 167 minutes, at the end of which time the pH of the working fluid was 5.5. The working fluid was then rinsed out of the cell with deionized water, layered with 60 ml. of ethyl acetate, and adjusted to

pH 1.7 with concentrated sulfuric acid. The aqueous layer was extracted again with an additional 60 ml. of ethyl acetate, and the combined organic layers were dried over sodium sulfate and evaporated to a solid under vacuum to obtain 330 mg. of impure product.

The following preparation illustrates the use of this invention for the synthesis of useful oxazolinoazetidinones.

Preparation 7

Diphenylmethyl 3-chloromethyl-2-[3-(4-methylphenyl)-7-oxo-epi-4-oxa-2,6-diazabicyclo[3,2,0]-hept-2-ene-6-yl]-3-butenoate

A 150 mg. portion of diphenylmethyl 7-α-(4-methylphenylformamido)-3-exomethylenecephem-4-carboxylate was dissolved in 20 ml. of dichloromethane, which had been stabilized with cyclohexane and dried over molecular sieves. The solution was chilled in a dry ice-acetone bath to about -78°, and 0.33 ml. of 1-molar chlorine gas in dichloromethane was added. The mixture was stirred at constant temperature for 45 minutes, and 86 mg. of triphenyl phosphine was added. The cooling bath was then removed, and the solution was stirred for two hours. The volatiles were evaporated away under vacuum, and the gummy residue was purified by chromatography over 3.5 g. of silica gel under about 5 psi of nitrogen pressure, eluting with a gradient solvent ranging from pure toluene to 5% ethyl acetate in toluene. The product-containing fractions were combined and evaporated to obtain 60 mg. of the desired

product, which was identified by nuclear magnetic resonance analysis in $CDCl_3$, using trimethylsilane as the internal standard.
δ2.37 (s, 3H, $CH_3$); 4.07 (s, 2H, $CH_2Cl$); 5.03, 5.20, 5.45 (s, 3H, = $CH_2$ and H4); 5.22, 5.87 (d, J = 4Hz, 2H, β-lactam); 6.80 (s, 1H, C<u>H</u>$Ph_2$); 7.08, 7.66 (AB, J = 8Hz, aromatic of amide); 7.20 (s, 10H, aromatic of ester).

0082656



## CLAIMS

1. A process for preparing a 7-*epi*-exomethylenecepham of the formula (I):

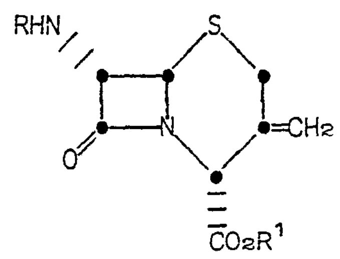

(I)

wherein R is hydrogen or an acyl group, and $R^1$ is hydrogen, a carboxy-protecting group, or a cation which process comprises reducing a sulfoxide of formula (II):

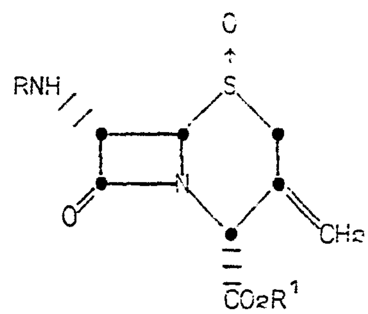

(II)

or acetoxymethyl derivative of formula (III):

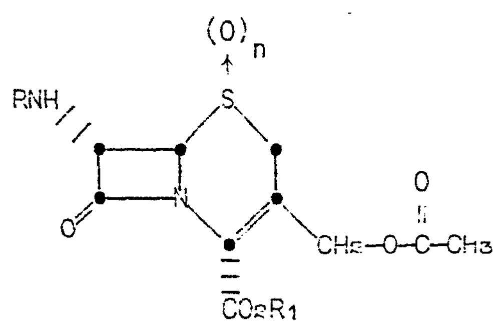

(III)

where n is 0 or 1 and R and $R^1$ are as defined above.

2. A process according to claim 1 in which the reduction of the sulfoxide of formula (II) is effected by electrolytic means or by reaction with an acyl halide and a $C_2$-$C_5$ alkene.

3. A process according to claim 2, wherein the acyl halide is a $C_2$-$C_4$ alkanoyl halide.

4. A process according to claim 1 in which the acetoxymethyl derivative of formula (III) is electrolytically reduced.

5. A process according to any one of claims 1 to 4, in which R is hydrogen or the formula $R^2$OC, in which $R^2$ is hydrogen, $C_1$-$C_3$ alkyl, cyanomethyl, benzyloxy, 4-nitrobenzyloxy, t-butoxy, 2,2,2-trichloroethoxy, 4-methoxybenzyloxy, or the group $R^3$, in which $R^3$ is phenyl or phenyl substituted by 1 or 2 halogen, protected hydroxy, nitro, cyano, trifluoromethyl, $C_1$-$C_4$ alkyl or $C_1$-$C_4$ alkoxy groups; or $R^2$ is a group of the formula $R^4-(O)_n-CH_2-$, in which $R^4$ has the same meanings as $R^3$ above, or is 1,4-cyclohexadienyl, 2-thienyl or 3-thienyl; n is 0 or 1; provided that when n is 1, $R^4$ has the same meanings as $R^3$; or $R^2$ is a group of the formula $R^4$-CH(W)-, in which $R^4$ has the same meanings as defined above, and W is protected hydroxy or protected amino.

6. A process according to claim 5 wherein $R^2$ is $C_1$-$C_3$ alkyl, phenyl, phenoxy, benzyl or phenyl substituted with $C_1$-$C_4$ alkyl.

7. A process according to any one of claims 1 to 6 in which $R^1$ is acetoxymethyl, 1-acetoxyethyl, pivaloyloxymethyl, 1-pivaloyloxyethyl, carboethoxyoxymethyl, 1-carboxyethoxyoxyethyl, phthalidyl, diphenylmethyl, nitrobenzyl, tert-butyl, methoxybenzyl, trichloroethyl, or methyl.

8. A compound of formula (I) whenever prepared by a process according to any one of claims 1 to 7.

9. A compound of formula (I) as defined in claim 1.

10. A process for preparing an antibiotically active oxa-β-lactam which comprises preparing a 7-epi-exomethylenecepham according to claim 1, and converting the 7-epi-exomethylenecepham into the antibiotically active oxa-β-lactam.